# EUROPEAN PATENT SPECIFICATION

(11) **EP 1 207 159 B1**
(45) Date of publication and mention of the grant of the patent: **16.06.2004**
(21) Application number: 02003994.7
(22) Date of filing: 18.03.1998
(51) Int. Cl.: C07D 401/06, C07D 417/06, A61K 31/445, A61K 31/505, A61P 25/18

(54) **Aromaheterocyclic derivatives as dopamine D4 receptor antagonists**
Aromaheterozyklische Verbindungen zur Verwendung als Dopamin-D4-rezeptor-antagonisten
Dérivés aromahétérocycliques pour utilisation comme antagonistes de dopamin-d4

(43) Date of publication of application: 22.05.2002
(62) Divisional of application: 98909749.8
(73) Proprietor: TAISHO PHARMACEUTICAL CO. LTD, Tokyo 171 (JP); Nihon Nohyaku Co., Ltd., Tokyo 103-8236 (JP)
(72) Inventor: Nakazato, Atsuro, c/o Taisho Pharmaceutical Co., Tokyo (JP); Kumagai, Toshihito, c/o Taisho Pharmaceutical Co., Tokyo (JP); Chaki, Shigeyuki, c/o Taisho Pharmaceutical Co., Tokyo (JP); Tomisawa, Kazuyuki, c/o Taisho Pharmaceutical Co., Tokyo (JP); Nagamine, Masashi, c/o Research Center of Nihon, Kawachinagano-shi, Osaka (JP); Gotoh, Makoto, c/o Research Center of Nihon, Kawachinagano-shi, Osaka (JP); Yoshida, Masanori, c/o Research Center of Nihon, Kawachinagano-shi, Osaka (JP)
(74) Representative: Grünecker, Kinkeldey, Stockmair & Schwanhäusser Anwaltssozietät

(56) References cited:
- EP-A- 0 816 362
- Thornber C.W.: 'Isoterism and Molecular Modification in Drug Design', Chemical Society Reviews, 1979, 8(4), p. 563-580

## Description

The present invention relates to dopamine D₄ receptor antagonist compounds useful as antipsychotic drugs.

Antipsychotic agents are used not only for treating schizophrenia and problem behaviors associated with cerebrovascular diseases and senile dementia (e.g. aggressive behaviors, psychogenic excitement, ecdemomania or delirium). However, dopamine D₂ receptor antagonists, prior art antipsychotic drugs, cause serious extrapyramidal diseases as side effects, which has been a serious problem.

On the other hand, recently found dopamine D₄ receptors are similar to dopamine D₂ receptors in structure and properties, but are utterly different from dopamine D₂ receptors in intracerebral distributions. The intracerebral distribution of dopamine D₄ receptors is such that they are present in a high concentrations in the corticocerebral frontal lobe concerned with the onset of schizophrenia and are present in a low concentration in the striatum concerned with the onset of extrapyramidal diseases. Accordingly, unlike dopamine D₂ receptor antagonists, dopamine D₄ receptor antagonists are very likely to become novel therapeutic agents of schizophrenia without extrapyramidal diseases as side effects (Nature, 350, 610-614 (1991); Nature, 358, 109 (1992); Nature, 365, 393 (1993); Nature, 365, 441-445 (1993)).

Among such compounds is included clozapine. It has been reported that the affinity of clozapine for dopamine D₄ receptors is higher than that for dopamine D₂ receptors (Nature, 350, 610-614 (1991)). It has also been reported that in a clinical investigation of clozapine, unlike dopamine D₂ receptor, clozapine is effective on drug-resistant schizophrenia and negativism and hardly causes extrapyramidal diseases (Arch. Gen. Psych., 45, 789-796 (1988)). However, clozapine has a serious defect of causing blood disorder called agranulocytosis, and cases of death therefrom have been reported (Summary and Clinical Data, Sandoz, Canada Inc. (1990)).

Accordingly, dopamine D₄ receptor antagonists without such side-effects are very useful as therapeutic agents of schizophrenia which are very unlikely to cause extrapyramidal diseases.

EP-A-816 362 discloses thiazole derivatives which have a high affinity for dopamine D4 receptors and which show an antipsychotic effect without any extrapyramidal disorder.

It is the object of the present invention to provide dopamine D₄ receptor antagonist compounds which have an antipsychotic action without causing extrapyramidal diseases.

Said object has been solved by an aromaheterocyclic derivative represented by Formula [I]: 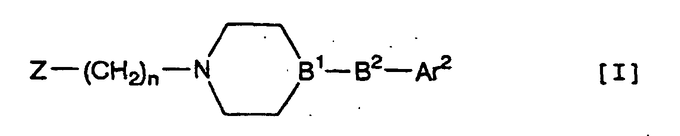 wherein Z is a group represented by the following Formula [III]; 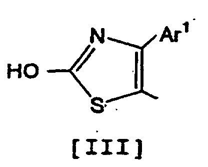 wherein Ar¹ is a phenyl group or a phenyl group substituted with a halogen atom or an alkyl group of 1 to 5 carbon atoms, Ar² is a phenyl group of one or two substituents selected from the group consisting of a halogen atom, an alkyl group of 1 to 5 carbon atoms, an alkoxy group of 1 to 5 carbon atoms, a hydroxyl group and a trifluoromethyl group, or a phenyl group, B¹-B² is CH-CO- or C=C(R¹) (wherein R¹ is a hydrogen atom or an alkyl group of 1 to 5 carbon atoms), and n is an integer of 1 to 4; or a pharmaceutically acceptable salt thereof.

In the present invention, the alkyl group of 1 to 5 carbon atoms refers to a straight or branched alkyl group, and examples thereof are a methyl group, an ethyl group, a propyl group, an isopropyl group, a butyl group and an isobutyl group. The alkylthio group of 1 to 5 carbon atoms refers to a straight or branched alkylthio group, and examples thereof are a methylthio group, an ethylthio group, a propylthio group, an isopropylthio group, a butylthio group and an isobutylthio group. The halogen atom refers to a fluorine atom, a chlorine atom, a bromine atom or an iodine atom. Accordingly, examples of the phenyl group of one or two substituents selected from the group consisting of a halogen atom, an alkyl group of 1 to 5 carbon atoms, an alkoxy group of 1 to 5 carbon atoms, a hydroxyl group and a trifluoromethyl group are a 2-fluorophenyl group, a 3-fluorophenyl group, a 4-fluorophenyl group, a 4-chlorophenyl group, a 4-bromophenyl group, a 3,4-dichlorophenyl group, a 4-methylphenyl group, a 3-trifluoromethylphenyl group, a 4-methoxyphenyl group, a 3,4-dimethoxyphenyl group and a 4-hydroxyphenyl group.

In addition, the pharmaceutically acceptable salt in the present invention includes salts with mineral acids such as sulfuric acid, hydrochloric acid or phosphoric acid, and salts with organic acids such as acetic acid, oxalic acid, lactic acid, tartaric acid, fumaric acid, maleic acid, trifluoroacetic acid or methanesulfonic acid.

Preferred compounds in the present invention can be indicated as follows:

That is, when Z is a group represented by Formula [III] in Formula [I], preferred compounds are those wherein Ar¹ is a fluorophenyl group, Ar² is a phenyl group substituted with a halogen atom, B¹-B² is CH-CO or C=CH and n is 2, and especially preferred compounds are those wherein Ar² is a fluorophenyl group and B¹-B² is C=CH.

The compound of Formula [I] can be prepared by the following methods. In the following reaction formulae, Ar¹, Ar², R¹, B¹-B² and n are as defined above, R⁶ is a protecting group of the nitrogen atom such as, for example, an alkoxycarbonyl group (e.g. a t-butoxycarbonyl group or an ethoxycarbonyl group), an acyl group (e.g. an acetyl group or a benzoyl group), a sulfonyl group (e.g. a tosyl group), an alkyl group of 1 to 5 carbon atoms or a benzyl group, R⁷ is an alkyl group of 1 to 5 carbon atoms, M is, for example, a sodium atom, a potassium atom or NH₄, X¹ is a chlorine atom, a bromine atom or an iodine atom.

### <Preparation Method 1>

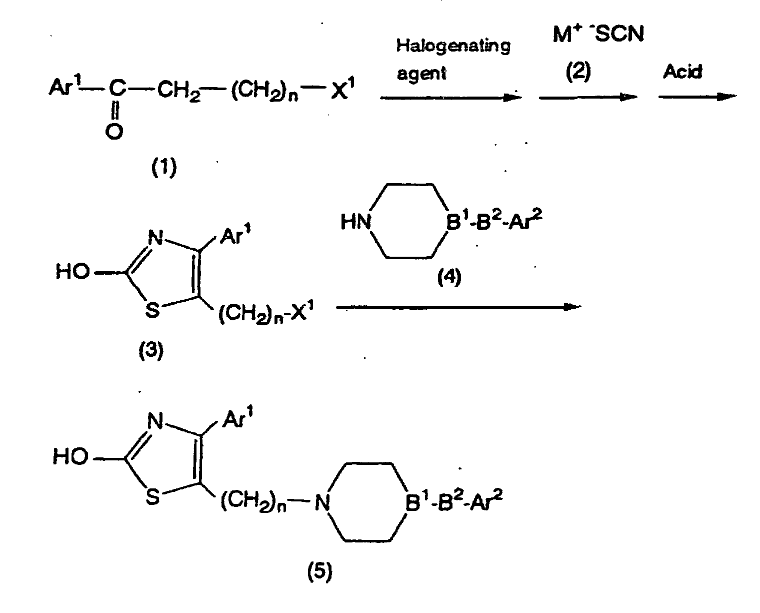

A ketone derivative (1) is halogenated with a halogenating agent in an inert solvent, and reacted with a thiocyanate (2) in an inert solvent, followed by an acid treatment to give 2-hydroxythiazole derivative (3).

The inert solvent includes organic carboxylic acids (e.g. acetic acid), organic halogenides (e.g. carbon tetrachloride or chloroform), alcohols (e.g. ethanol or isopropanol), ethers (e.g. diethyl ether or tetrahydrofuran), hydrocarbons (e.g. toluene), N,N-dimethylformamide, acetonitrile, water or a mixture thereof. Examples of the halogenating agent are chlorine, bromine, iodine, N-chlorosuccinimide, N-bromosuccinimide and sulfuryl chloride. The acid treatment refers to a reaction with an acid such as hydrochloric acid, sulfuric acid, acetic acid, trifluoroacetic acid, methansulfonic acid, tosylic acid or a mixture thereof in an alcohol (e.g. methanol or ethanol), an ether (e.g. dioxane), acetone or water.

Subsequently, the 2-hydroxythiazole derivative (3) is reacted with an amine (4) in the presence or absence of a base in an inert solvent to give a compound (5) of the present invention.

Examples of the base are organic amines(e.g. triethylamine, N,N-diisopropylethylamine or pyridine), alcoholates (e.g. sodium ethoxide), alkali metal amides (e.g. sodium amide), inorganic bases (e.g. sodium bicarbonate, sodium carbonate, potassium carbonate, sodium hydroxide or sodium hydride). Examples of the inert solvent are organic halogenides (e.g. carbon tetrachloride or chloroform), alcohols (e.g. methanol, ethanol or isopropanol), ethers (e.g. diethyl ether or tetrahydrofuran), hydrocarbons (e.g. toluene), N,N-dimethylformamide, acetonitrile, water and a mixture thereof.

The amine (4) can be prepared according to the method described below.

### <Preparation Method 2>

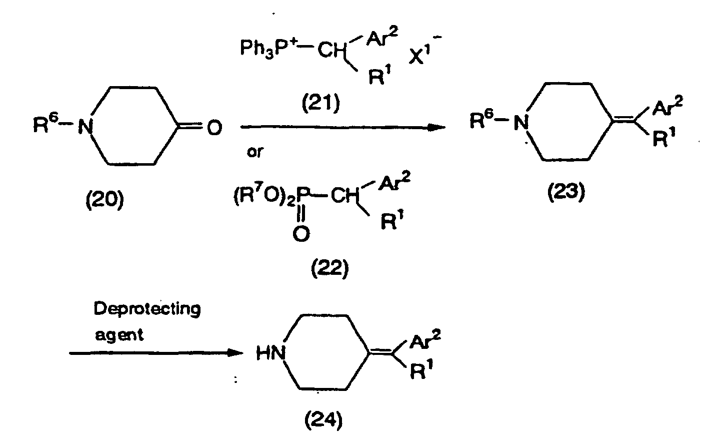

A 4-benzylidenepiperidine compound (24) can be prepared by condensing a piperidone derivative (20) with a triphenylarylmethyl phosphonium salt (21) or a dialkylarylmethyl phosphonate (22) in the presence of a base in an inert solvent, and then removing the protective group with deprotecting agent.

Examples of the base are sodium hydride, potassium hydride, sodium methoxide, potassium t-butoxide, n-butyl lithium, lithium diisopropylamide, lithium bis(trimethylsilyl)amide and sodium amide, and if necessary, they can be used together with a catalyst (e.g. 15-crown-5 ether or 18-crown-6 ether), tetramethylethylenediamine or hexamethylphosphoramide. Examples of the inert solvent are ethers (e.g. diethyl ether, tetrahydrofuran or dioxane), hydrocarbons (e.g. benzene or toluene), alcohols (e.g. ethanol), N,N-dimethylformamide, dimethylsulfoxide, water and a mixture thereof. Examples of a reaction solvent to be used for the deprotection are ethers (e.g. diethyl ether, tetrahydrofuran or dioxane), hydrocarbons (e.g. benzene or toluene), alcohols (e.g. ethanol), organic carboxylates (e.g. ethyl acetate), ketones (e.g. acetone), halogenated alkanes (dichloromethane or chloroform), organic carboxylic acids (e.g. acetic acid), N,N-dimethylformamide and water. Examples of the deprotecting agent, in case where R⁶ is an alkoxycarbonyl' group, an acyl group or a sulfonyl group, are acids such as inorganic acids (e.g. hydrochloric acid, hydrobromic acid or sulfuric acid), organic acids (e.g. trifluoroacetic acid, formic acid or methanesulfonic acid) or a solution of hydrogen chloride in dioxane or ethyl acetate, and bases such as inorganic bases (e.g. sodium hydroxide, potassium hydroxide or barium hydroxide). In case where R⁶ is an alkyl group of 1 to 5 carbon atoms or a benzyl group, after conversion into an alkoxycarbonyl group according to a reaction with an alkyl haloformate (e.g. ethyl chloroformate) in the presence or absence of a base, deprotection is carried out in the same manner as described above. In case where R⁶ is a benzyl group, deprotection can be carried out according to a Birch reduction.

The compounds of the present invention show a superior affinity for dopamine D₄ receptor but do not show a low affinity for dopamine D₂ receptor, and have a superior selectivity. Accordingly, the compounds of the present invention are useful for the prevention or treatment of problem behaviors associated with cerebrovascular diseases and senile dementia, and useful as drugs without extrapyramidal diseases as side-effects.

For the above-mentioned purposes, the compounds of the present invention are combined with conventional fillers, binding agents, disintegrators, pH modulators, solubilizers, etc. to formulate into tablets, pills, granules, powders, solutions, emulsions, suspensions,' injections, etc. according to conventional preparation techniques.

The compound of the present invention can be administered orally or parenterally in the dose of from 0.1 to 500 mg/day to an adult patient in a single portion or several divided portions. This dose can be properly increased or decreased on the type of diseases, the age, body weight or symptoms of the patient.

The present invention is illustrated in more detail by showing the following examples and experiments.

### Example 1

### Synthesis of 2-hydroxy-5-(2-chloroethyl)-4-(4-fluorophenyl)thiazole

To a solution of 20.08 g of 4-chloro-4'-fluorobutyrophenone in 80 ml of chloroform was added dropwise a solution of 5.2 ml of bromine in 10 ml of chloroform over 30 minutes. The reaction mixture was stirred at room temperature for an hour, and concentrated under reduced pressure.

The residue was dissolved in 120 ml of ethanol, and 9.80 g of potassium thiocyanate was added, followed by reflux with heating with stirring for an hour. The reaction solution was concentrated under reduced pressure, and to the residue was added water, followed by extracting with ethyl acetate. The extract was washed with water and a saturated aqueous sodium chloride solution, dried over anhydrous sodium sulfate, and the drying agent was removed by filtration, followed by concentration under reduced pressure.

The residue was refluxed in a mixture of 140 ml of acetic acid, 40 ml of water and 15 ml of sulfuric acid with heating with stirring for 3 hours. The reaction solution was concentrated under reduced pressure, and the residue was poured into ice water and extracted with ethyl acetate. The extract was washed with water, a saturated aqueous sodium bicarbonate solution and a saturated aqueous sodium chloride solution and dried over anhydrous sodium sulfate, and after removal of the drying agent by filtration, the filtrate was concentrated under reduced pressure. The residue was crystallized by addition of isopropyl ether, and recrystallized from hexane-ethyl acetate to give 16.40 g of 2-hydroxy-5-(2-chloroethyl)-4-(4-fluorophenyl)thiazole.
m.p. 140.0 - 141.5°C

### Example 2

### Synthesis of 2-hydroxy-4-(4-fluorophenyl)-5-[2-[4-(3-fluorobenzylidene)piperidin-1-yl]ethyl]thiazole

In 2 ml of methanol were stirred 773 mg of 2-hydroxy-5-(2-chloroethyl)-4-(4-fluorophenyl)thiazole, 683 mg of 4-(3-fluorobenzylidene)piperidine hydrochloride and 1.04 ml of N,N-diisopropylethylamine at 80°C for 3 days. The reaction solution was concentrated under reduced pressure, and the residue was separated with ethyl acetate and a saturated aqueous sodium bicarbonate solution. The organic layer was washed with a saturated aqueous sodium chloride solution and dried over anhydrous sodium sulfate, and the drying agent was removed by filtration. The filtrate was concentrated under reduced pressure, and the residue was purified by a flash column chromatography (silica gel; ChromatorexNH NHDM1020 (manufactured by Fuji-Davison Chemical Co.), eluent; hexane-ethyl acetate =1:1), and recrystallized from hexane-ethyl acetate to give 265 mg of 2-hydroxy-4-(4-fluorophenyl)-5-[2-[4-(3-fluorobenzylidene)piperidin-1-yl]ethyl]thiazole.
m.p. 140.5 - 142.0°C

The structures and physical property data of the present compound and the compounds prepared in the same manner as in Example 2 are shown in Table 1.

### Example 3

### Synthesis of 4-(4-fluorobenzylidene)piperidine hydrochloride

To a stirred suspension of 13.20 g of 60 % sodium hydride (in oil) containing 1.65 g of 15-crown-5 ether in 650 ml of tetrahydrofuran were added dropwise a solution of 59.78 g of N-t-butoxycarbonylpiperidone and 81.25 g of diethyl 4-fluorobenzylphosphonate in 150 ml of tetrahydrofuran under ice-cooling over 20 minutes. After stirring at room temperature for a day, a saturated aqueous sodium bicarbonate solution was added cautiously, followed by extracting with ethyl acetate. The extract was washed with a saturated aqueous sodium bicarbonate solution and a saturated aqueous sodium chloride solution, successively, and dried over anhydrous sodium sulfate, followed by removal of the drying agent by filtration. The filtrate was concentrated under reduced pressure and purified by a flash column chromatography (silica gel: Wakogel C200 (manufactured by Wako Pure Chemicals), eluent; hexane-ethyl acetate =20:1) to give 55.23 g of N-t-butoxycarbonyl-4-(4-fluorobenzylidene)piperidine as an oil, which was then crystallized by allowing to stand at room temperature overnight.
m.p. 69 - 70°C

To 55.00 g of N-t-butoxycarbonyl-4-(4-fluorobenzylidene)piperidine was added 475 ml of an ice-cooled solution of 4 N hydrogen chloride in dioxane, followed by stirring at room temperature for 2 hours. The reaction solution was concentrated under reduced pressure, and the resulting crystals were recrystallized from isopropanol to give 40.72 g of 4-(4-fluorobenzylidene)piperidine hydrochloride.
m.p. 184 - 185.5°C

The structures and physical property data of the present compound and the compounds prepared in the same manner as in Example 3 are shown in Table 2.

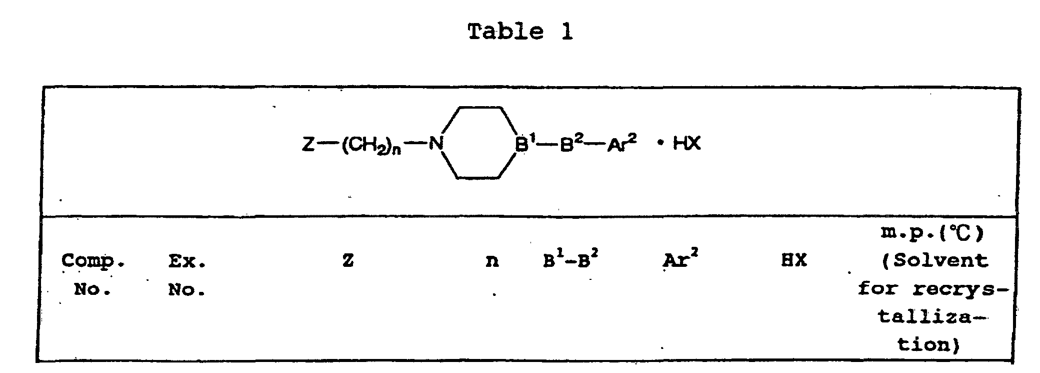

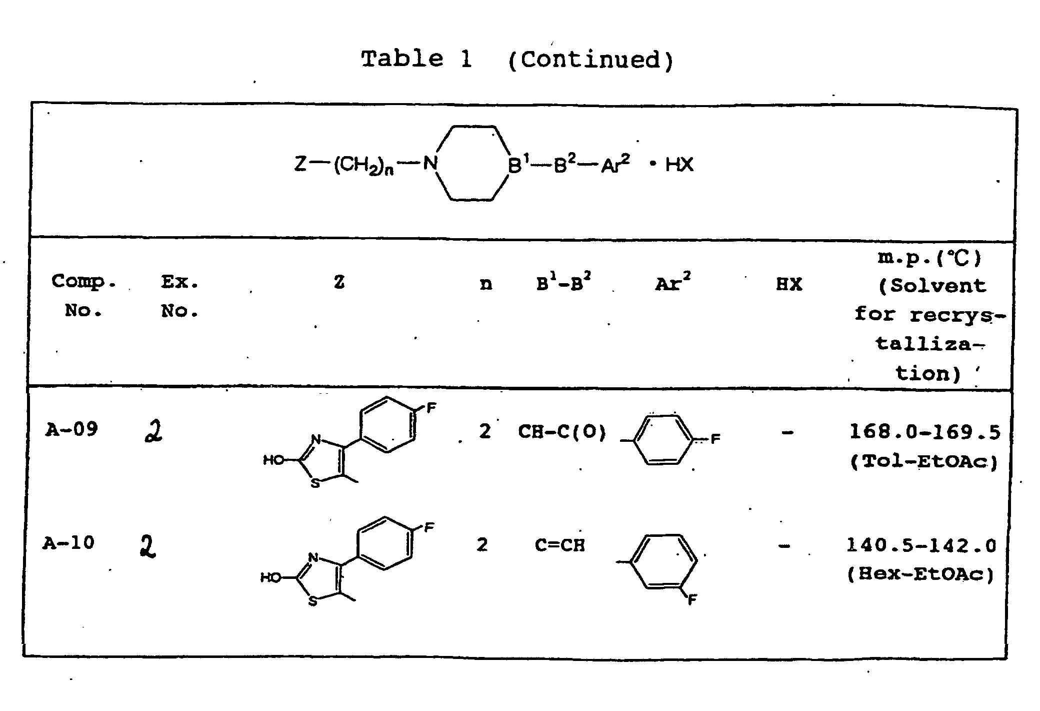

In Table 1,
Comp. No.; Compound Number
Ex. No.; Example Number used for synthesis of the compound.

Solvent for recrystallization; IPE = diisopropyl ether, IPA = isopropyl alcohol, Hex = hexane, EtOAc = ethyl acetate, Tol = toluene, EtOH = ethanol, MeOH = methanol

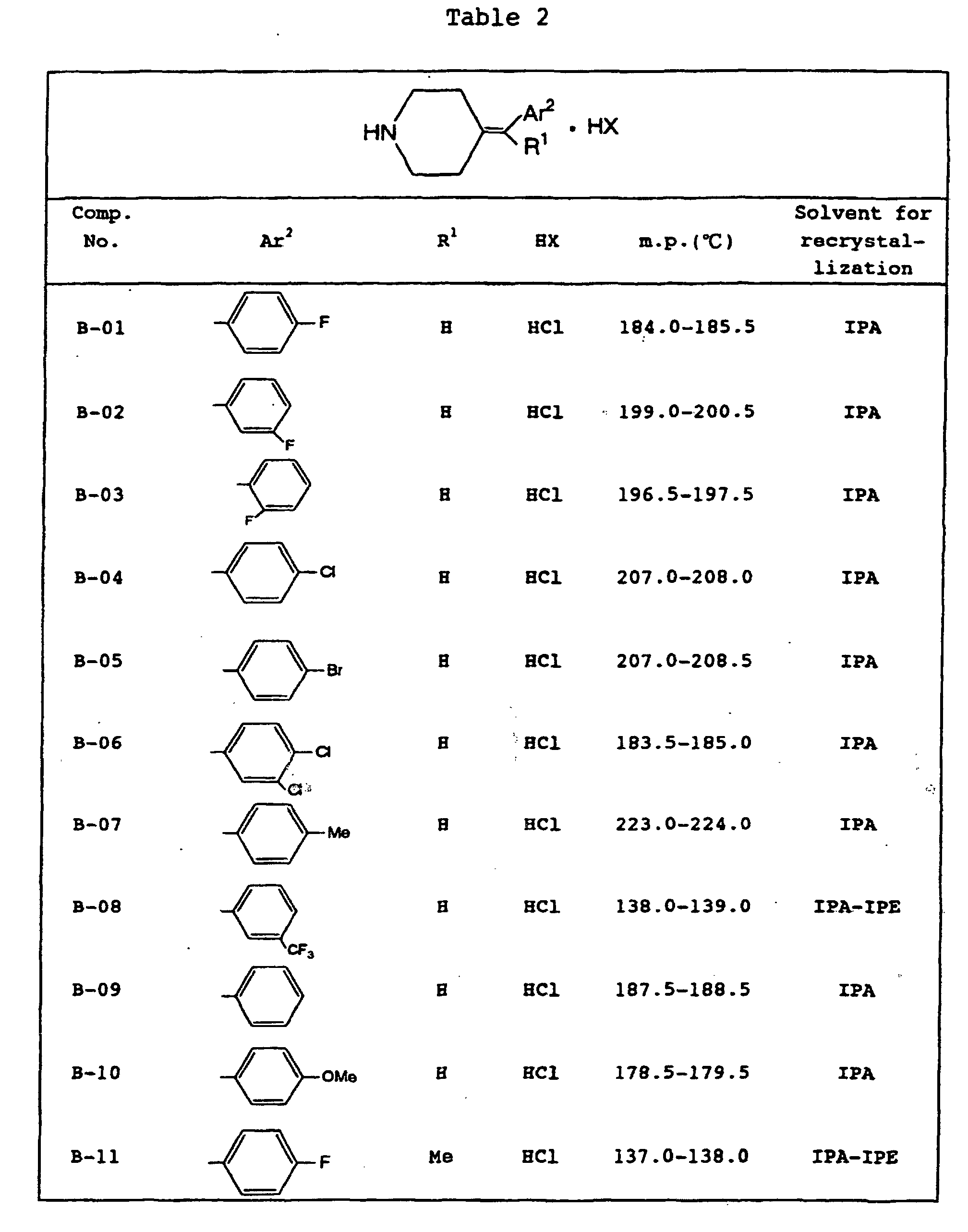

In Table 2,
Comp. No.; Compound Number
Solvent for recrystallization ; IPA isopropyl alcohol, IPE = diisopropyl ether.

### Experiment [Receptor Binding Assay]

### 1. Dopamine D₄ Receptor Binding Assay

Chinese hamster ovarium (CHO) cell membranes wherein human D_{4.2} receptor was expressed were used as a receptor preparation.

[³H]spiperone was used as [³H]-labeled ligand.

A binding reaction using the [³H]-labeled ligand was carried out according to the following method as described in Eur. J. Pharmacol., 233, 173(1993).

Dopamine D_{4.2} Receptor Binding Assay: The CHO cell membranes wherein human D_{4.2} receptor was expressed, [³H]spiperone (0.5 nM) and each test drug were reacted in 50 mM Tris-hydrochloric acid buffer (pH 7.4) containing 5 mM EDTA, 1.5 mM CaCl₂, 5 mM KCl and 120 mM NaCl at 27°C for 2 hours.

After completion of the reaction, the reaction solution was filtered by suction through a glass filter (GF/B), and the radioactivity on the filter was measured by a liquid scintillation spectrometer.

The binding under the reaction in the presence of 10 µM haloperidol was defined as non-specific binding of [³H]spiperone, and the difference between the total binding and the non-specific binding was defined as specific binding. An inhibition curve was obtained by reacting a definite concentration of [³H]spiperone with various concentrations of test drug under the above-mentioned conditions, and the concentration (IC₅₀) of the test drug to exhibit 50% inhibition of [³H]spiperone binding was determined by the inhibition curve. The results are shown in Table 3.

### 2. Dopamine D₂ Receptor Binding Assay

Rat striatum membrane was used as a receptor preparation.

[³H]raclopride was used as [³H]-labeled ligand.

A binding reaction using the [³H]-labeled ligand was carried out according to the following method as described in Mol. J. Pharmacol., 43, 749(1993).

Preparation of Receptor Preparation: Rat striatum was homogenized in 50 mM Tris-hydrochloric acid buffer (pH 7.4), centrifuged at 48,000 × g, and the precipitate was washed once with a Tris-hydrochloric acid buffer. The precipitate was suspended in 50 mM Tris-hydrochloric acid buffer (pH 7.4) containing 120 mM NaCl, 1.5 mM KCl, 2 mM CaCl₂ and 1 mM MgCl₂ to give a membrane preparation.

Dopamine D₂ Receptor Binding Assay: The membrane preparation (0.5 mg of protein/ml), [³H]raclopride (1 nM) and each test drug were reacted at 25°C for an hour.

After completion of the reaction, the reaction solution was filtered by suction through a glass filter (GF/B), and the radioactivity on the filter was determined by a liquid scintillation spectrometer.

The binding under the reaction in the presence of 10 µM haloperidol was defined as non-specific binding of [³H]raclopride, and the difference between total binding and non-specific binding was defined as specific binding. An inhibition curve was obtained by reacting a definite concentration of [³H]raclopride with varied concentrations of the test drug under the above-mentioned conditions, and the concentration (IC₅₀) of the test drug to exhibit 50% inhibition of [³H]raclopride binding was determined by the inhibition curve. The results are shown in Table 3.

**Table 3**

| Compound No. | IC₅₀ (nM) | |
|---|---|---|
| | D₄ | D₂ |
| A-09 | 2.85 | 107.2 |
| A-10 | 1.96 | 73.9 |

### Industrial Applicability

The compounds of the present invention are useful for the prevention or the treatment of schizophrenia and problem behaviors associated with cerebrovascular diseases or senile dementia, and they are highly effective when administered orally, and are useful as drugs without extrapyramidal diseases as side-effects.

## Claims

1. An aromaheterocyclic derivative represented by formula [I]: 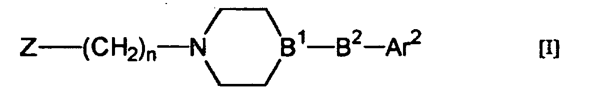 wherein Z is a group represented by the following formula [III]: 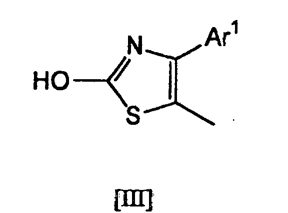 wherein
Ar¹ is a phenyl group or a phenyl group substituted with a halogen atom or an alkyl group of 1 to 5 carbon atoms;
Ar² is a phenyl group having one or two substituents selected from the group consisting of a halogen atom, an alkyl group of 1 to 5 carbon atoms, an alkoxy group of 1 to 5 carbon atoms, a hydroxyl group and a trifluoromethyl group, or a phenyl group;
B¹-B² is CH-CO or C=C(R¹) wherein R¹ is a hydrogen atom or an alkyl group of 1 to 5 carbon atoms;
n is an integer of 1 to 4;
or pharmaceutically acceptable salts thereof.

2. The aromaheterocyclic derivative according to claim 1, wherein Z is a group represented by formula [III], Ar¹ is a fluorophenyl group, Ar² is a phenyl group substituted with a halogen atom, B¹-B² is CH-CO or C=CH and n is 2 in formula [I]; or pharmaceutically acceptable salts thereof.

3. A dopamine D₄ receptor antagonist preparation which comprises an aromaheterocyclic derivative according to claim 1 or 2 as an effective ingredient.

4. Use of the aromaheterocyclic derivative claim 1 or 2 for the manufacture of a medicament for the treatment of psychotic behaviour.

## Patentansprüche

1. Aromatisches heterocyclisches Derivat, das durch die Formel [I] wiedergegeben wird: 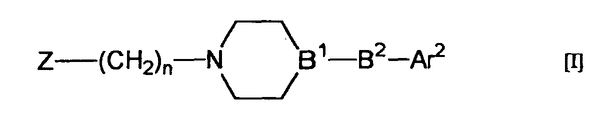 worin Z eine Gruppe ist, die durch die folgende Formel [III] wiedergegeben wird: 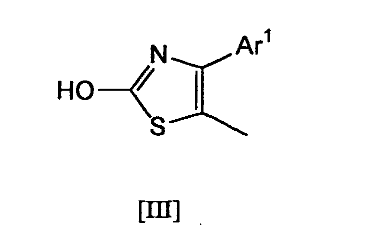 worin Ar¹ eine Phenylgruppe oder eine mit einem Halogenatom oder einer Alkylgruppe mit 1 bis 5 Kohlenstoffatomen substituierte Phenylgruppe ist,
Ar² eine Phenylgruppe mit einem oder zwei Substituenten, ausgewählt aus der Gruppe bestehend aus einem Halogenatom, einer Alkylgruppe mit 1 bis 5 Kohlenstoffatomen, einer Alkoxygruppe mit 1 bis 5 Kohlenstoffatomen, einer Hydroxylgruppe und einer Trifluormethylgruppe, oder eine Phenylgruppe ist,
B¹-B² CH-CO oder C=C(R¹) ist, wobei R¹ ein Wasserstoffatom oder eine Alkylgruppe mit 1 bis 5 Kohlenstoffatomen ist,
n eine ganze Zahl von 1 bis 4 ist;
oder ein pharmazeutisch annehmbares Salz davon.

2. Aromatisches heterocyclisches Derivat nach Anspruch 1, wobei Z eine Gruppe ist, die durch die Formel [III] wiedergegeben wird, Ar¹ eine Fluorphenylgruppe ist, Ar² eine mit einem Halogenatom substituierte Phenylgruppe ist, B¹-B² CH-CO oder C=CH ist und n in der Formel [I] 2 ist; oder das pharmazeutisch annehmbare Salz davon.

3. Dopamin D₄-Rezeptorantagonisten-Zubereitung, welche das aromatische heterocyclische Derivat nach Anspruch 1 oder 2 als Wirkstoff umfasst.

4. Verwendung des aromatischen heterocyclischen Derivats nach Anspruch 1 oder 2 zum Herstellen eines Arzneimittels für die Behandlung von psychotischem Verhalten.

## Revendications

1. Dérivé hétérocyclique aromatique représenté par la formule [I] : 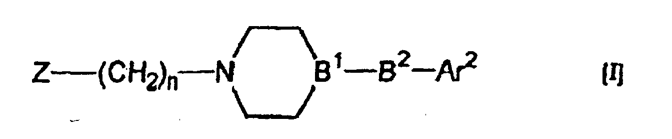 dans laquelle Z est un groupe représenté par la formule [III] suivante : 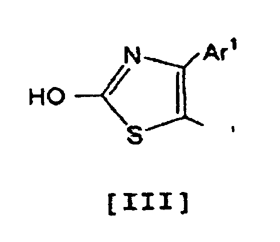 où
Ar¹ est un groupe phényle ou un groupe phényle substitué avec un atome d'halogène ou un groupe alkyle de 1 à 5 atomes de carbone;
Ar² est un groupe phényle ayant 1 ou 2 substituants choisis dans le groupe constitué par un atome d'halogène, un groupe alkyle de 1 à 5 atomes de carbone, un groupe alcoxy de 1 à 5 atomes de carbone, un groupe hydroxyle et un groupe trifluorométhyle, ou un groupe phényle;
B¹-B² est CH-CO ou C=C (R¹), où R¹ est un atome d'hydrogène ou un groupe alkyle de 1 à 5 atomes de carbone;
n est un nombre entier de 1 à 4;
ou sels pharmaceutiquement acceptables de celui-ci.

2. Dérivé hétérocyclique aromatique selon la revendication 1, dans lequel Z est un groupe représenté par la formule [III], Ar¹ est un groupe fluorophényle, Ar² est un groupe phényle substitué avec un atome d'halogène, B¹-B² est CH-CO ou C=CH et n est 2 dans la formule [I];
ou sels pharmaceutiquement acceptables de celui-ci.

3. Préparation d'antagoniste du récepteur D₄ de la dopamine qui comprend un dérivé hétérocyclique aromatique selon la revendication 1 ou 2 en tant qu'ingrédient actif.

4. Utilisation du dérivé hétérocyclique aromatique selon la revendication 1 ou 2 pour la fabrication d'un médicament pour le traitement d'un comportement psychotique.
